# EUROPEAN PATENT SPECIFICATION

(11) **EP 4 065 041 B1**
(45) Date of publication and mention of the grant of the patent: **30.07.2025**
(21) Application number: 21705703.3
(22) Date of filing: 13.01.2021
(51) Int. Cl.: A61F 2/01

(54) **FILTER FOR DEPLOYMENT SYSTEM**
FILTER FÜR EINSATZSYSTEM
FILTRE POUR SYSTÈME DE DÉPLOIEMENT

(30) Priority: 17.01.2020 US 202062962386 P
(43) Date of publication of application: 05.10.2022
(73) Proprietor: Edwards Lifesciences Corporation, Irvine, CA 92614-5688 (US)
(72) Inventor: LEE, Jeong Soo, Irvine, CA 92614 (US); REICH, Tal, 6037606 Or Yehuda (IL); GOLDBERG, Eran, 30889 Caesarea (IL)
(74) Representative: Venner, Julia Ann
(86) International application number: PCT/US2021/013170
(87) International publication number: WO 2021/146232

(56) References cited:
- US-A1- 2007 299 466
- US-A1- 2012 330 346
- US-A1- 2018 200 040

## Description

### CROSS-REFERENCE TO RELATED APPLICATIONS

This application claims the benefit of U.S. Provisional Application No. 62/962386, filed January 17, 2020.

### TECHNICAL FIELD

The present disclosure describes systems, devices, and methods related to implant deployment in fluidic systems and filtering mechanisms.

### BACKGROUND

A variety of maladies may affect an individual's body. Such maladies may be of the individual's heart, and may include maladies of the individual's heart valves, including the aortic, mitral, tricuspid, and pulmonary valves. Stenosis, for example, is a common and serious valve disease that may affect the operation of the heart valves and an individual's overall well-being.

Implants may be provided that may replace or repair portions of a subject's heart. Prosthetic implants, such as prosthetic heart valves, may be provided to replace portion of a subject's heart. Prosthetic aortic, mitral, tricuspid, and even pulmonary valves may be provided.

Implants may be deployed to the desired portion of the subject percutaneously, in a minimally invasive manner. Such deployment may occur transcatheter, in which a catheter may be deployed through the vasculature of an individual.

During deployment of such implants, care must be taken not to produce further maladies of the individual. For example, particles may be produced within the subject during deployment of such implants, which may cause maladies such as a stroke.

US 2007/0299466 describes a percutaneous transluminal angioplasty device including an embolic filter mounted to the catheter shaft at a location distal to the angioplasty balloon. Thus the filter is downstream from the blockage and is properly positioned to capture embolic particles that may be set loose into the blood stream as the angioplasty procedure is performed. The embolic filter is normally collapsed against the catheter shaft to facilitate introduction and withdrawal of the device to and from the operative site. Once the angioplasty balloon is properly positioned. however, means operatively associated with the embolic filter are actuated to erect the filter to position a filter mesh across the lumen of the vessel.

US 2012/0330346 describes an endoluminal catheterization device for providing protection against distal embolization of atherosclerotic debris and thrombi emboli resulting from an endoluminal catheterization procedure. The device is adapted to the new TAVI/PAVI methods to prevent the severe risk of brain embolization and stroke. The embolization protection device may also be an integral part of any other intra-luminal treatment or diagnostic device that may induce embolization, such as a balloon, stent. TAVI or atherectomy.

US 2018/0200040 describes an embolic protection system which may include a guidewire having a length, at least a portion of the guidewire having a non-circular cross-sectional shape, and an embolic protection device including a mounting sleeve configured to attach the embolic protection device to the portion of the guidewire having the non-circular cross-sectional shape. The mounting sleeve may include a non-circular interior cross-sectional shape and a non-circular exterior cross-sectional shape.

### SUMMARY

Aspects of the presently claimed invention are set out in the independent claims. Particular embodiments of these aspects are set out in the dependent claims. Any subject matter contained herein that does not fall within the scope of the appended claims is considered as being useful for understanding the invention.

The present systems, devices and methods relate to filtering particles within a subject in various procedures, including (but not limited to) medical and training procedures. Such filtering may occur as part of a deployment system for an implant within a subject. Subjects include (but are not limited to) medical patients, veterinary patients, animal models, cadavers, and simulators of the cardiac and vasculature system (e.g., anthropomorphic phantoms and explant tissue). In the methods of the presently claimed invention, the subject comprises a cadaver or simulator of the cardiac and vasculature system.

One aspect of the presently claimed invention provides a deployment system for deploying a filter in a subject. The deployment system includes a deployment apparatus including an elongate shaft having a deployment device.

The deployment system includes a filter body having a proximal portion and a distal portion, and configured to have a deployed state in which the filter body extends radially outward from the elongate shaft and increases in size from the proximal portion to the distal portion, the filter body configured to trap particles in the filter body.

The deployment system includes a filter support being positioned distal of the proximal portion of the filter body and configured to couple to the elongate shaft and slide relative to the filter body.

The deployment system includes one or more support tethers extending from the filter support to the distal portion of the filter body.

The deployment system includes a control device passing distally through the proximal portion of the filter body and coupling to the filter support, the control device configured to be slid proximally relative to the filter body to slide the filter support proximally to move the one or more support tethers and transition the filter body to the deployed state.

A second aspect of the presently claimed invention provides a method for deploying a filter in a subject. The method includes inserting a filter within a subject, the filter positioned upon an elongate shaft of a deployment apparatus having a deployment device.

The filter includes a filter body having a proximal portion and a distal portion, and configured to have a deployed state in which the filter body extends radially outward from the elongate shaft and increases in size from the proximal portion to the distal portion, the filter body configured to trap particles in the filter body, a filter support being positioned distal of the proximal portion of the filter body and on the elongate shaft and configured to slide relative to the filter body, and one or more support tethers extending from the filter support to the distal portion of the filter body.

The method includes sliding a control device, that passes distally through the proximal portion of the filter body and couples to the filter support, proximally to slide the filter support proximally to move the one or more support tethers and transition the filter body to the deployed state.

In embodiments of the disclosure herein, a deployment system for an implant may be provided. The deployment system may include a deployment apparatus including an elongate shaft having a deployment device. A filter may be configured to extend radially outward from the elongate shaft and configured to trap particles in the filter. An elongate sheath may be coupled to the filter and having a length and a cut extending along the length of the elongate sheath and an interior cavity configured to receive the elongate shaft of the deployment apparatus, the elongate sheath configured to couple to the elongate shaft of the deployment apparatus by the elongate shaft being passed through the cut of the elongate sheath to be positioned within the interior cavity of the elongate sheath.

In embodiments of the disclosure herein, a method may be provided. The method may include inserting an elongate shaft of a deployment apparatus into an interior cavity of an elongate sheath through a cut extending along a length of the elongate sheath, a distal portion of the elongate sheath being coupled to a filter. The method may include sliding the elongate sheath distally along the elongate shaft. The method may include deploying the filter within vasculature of a subject, the filter extending radially outward from the elongate shaft of the deployment apparatus and configured to trap particles in the filter.

In embodiments of the disclosure herein, a catheter system may be provided. The catheter system may include an expandable catheter sheath configured to be inserted into a vasculature and having an interior lumen configured for an apparatus to be passed through, the interior lumen having an interior diameter that is configured to increase upon the apparatus passing through the interior lumen. The catheter system may include a filter positioned at a distal portion of the expandable catheter sheath and configured to expand from an undeployed state radially outward to a deployed state in response to the apparatus passing through the interior lumen and applying a radial outward force to the interior lumen.

In embodiments of the disclosure herein, a method may be provided. The method may include passing an apparatus through an interior lumen of an expandable catheter sheath positioned within a subject. The method may include applying a radial outward force to the interior lumen with the apparatus to expand a filter positioned at a distal portion of the expandable catheter sheath from an undeployed state radially outward to a deployed state.

In embodiments of the disclosure herein, a catheter system may be provided. The catheter system may include an introducer sheath configured to be inserted into a vasculature and having a length and an interior lumen configured for an apparatus to be passed through. The catheter system may include a filter positioned at a central portion or a proximal portion of the introducer sheath and configured to extend radially outward from the introducer sheath to trap particles in the filter.

In embodiments herein of the disclosure, a method may be provided. The method may include inserting an introducer sheath into a vasculature, the introducer sheath including a filter positioned at a central portion or a proximal portion of the introducer sheath and configured to extend radially outward from the introducer sheath to trap particles in the filter.

### BRIEF DESCRIPTION OF THE DRAWINGS

These and other features, aspects, and advantages are described below with reference to the drawings, which are intended to illustrate, but not to limit, the disclosure. In the drawings, like reference characters denote corresponding features consistently throughout similar embodiments.
FIG. 1 is a side view of a deployment apparatus.
FIG. 2 is a perspective view of a prosthetic heart valve.
FIG. 3 is a side schematic view of a filter according to an embodiment of the present disclosure, with a portion of a retainer and control device shown in cross section.
FIG. 4 is a side schematic view of the filter shown in FIG. 3 in a deployed configuration.
FIG. 5 is a side perspective view of the filter shown in FIG. 3 in a deployed configuration.
FIG. 6 is a side schematic view of the filter shown in FIG. 3 in an undeployed configuration.
FIG. 7 is a cross sectional view along line 7-7 in FIG. 3.
FIG. 8 is a schematic view of a deployment apparatus approaching an aortic valve.
FIG. 9 is a schematic view of a filter deployed within a vasculature.
FIG. 10 is a schematic view of a deployed prosthetic aortic valve.
FIG. 11 is a side schematic view of a filter according to an embodiment of the present disclosure.
FIG. 12 is a side schematic view of a filter according to an embodiment of the presently claimed invention.
FIG. 13 is a side view of an introducer sheath according to an embodiment of the present disclosure.
FIG. 14 is a cross sectional view of a distal portion of the introducer sheath along line 14-14 in FIG. 13.
FIG. 15 is a cross sectional view of the distal portion of the introducer sheath shown in FIG. 13 with a filter deployed.
FIG. 16 is a schematic view of the introducer sheath shown in FIG. 13 passed towards an aortic valve.
FIG. 17 is a schematic view of the filter shown in FIG. 15 deployed.
FIG. 18 is a schematic view of a deployment apparatus extending through the introducer sheath shown in FIG. 17.
FIG. 19 is a cross sectional view of a distal portion of an introducer sheath according to an embodiment of the present disclosure.
FIG. 20 is a cross sectional view of an apparatus passing through the distal portion of the introducer sheath shown in FIG. 19.
FIG. 21 is a cross sectional view of the apparatus shown in FIG. 20 passing through the distal portion of the introducer sheath shown in FIG. 20.
FIG. 22 is a cross sectional view of an introducer sheath according to an embodiment of the present disclosure.
FIG. 23 is a side view of an introducer sheath according to an embodiment of the present disclosure.
FIG. 24 is a side cross sectional view of a filter of the introducer sheath along line 24-24 in FIG. 23.
FIG. 25 is a side perspective view of the handle of the introducer sheath shown in FIG. 23 positioned exterior to a skin.
FIG. 26 is a cross sectional schematic view of the introducer sheath shown in FIG. 23 extending into a subject's artery through the skin.
FIG. 27 is a cross sectional schematic view of the introducer sheath shown in FIG. 26 retracted from the subject's artery and out the skin.
FIG. 28 is a cross sectional view of an introducer sheath according to an embodiment of the present disclosure.

### DETAILED DESCRIPTION

The following description and examples illustrate some example embodiments of the disclosure in detail. Those of skill in the art will recognize that there are numerous variations and modifications of the disclosure that are encompassed by its scope. Accordingly, the description of a certain example embodiment should not be deemed to limit the scope of the present disclosure.

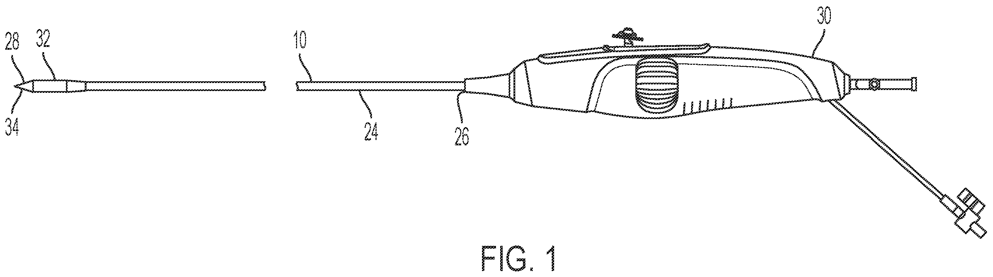

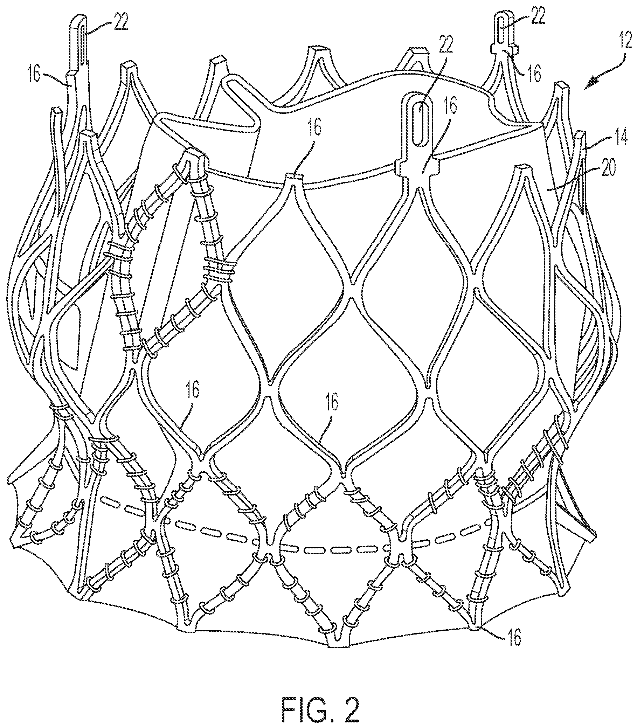

FIG. 1 illustrates an embodiment of a deployment apparatus 10 that may be utilized to deploy an implant to a portion of a subject according to embodiments disclosed herein. The implant 12 as marked in FIG. 2 may be an aortic implant comprising a prosthetic aortic valve. In embodiments, the implant may have other forms than shown in FIG. 2, for example the implant may be a mitral, tricuspid, or pulmonary prosthetic valve, among other forms of prosthetics. The implant may comprise a stent, clip, or other form of implant that may be inserted in a portion of the subject, including, in some instances, the heart of a patient.

The implant 12 may be an expandable implant as shown in FIG. 2, which may be configured to be expanded to be placed in position within the native valve location. The implant 12 may include a frame 14 including a plurality of supports 16 configured to be compressed for positioning within the deployment apparatus 10 and configured to be expanded at the desired time. The frame 14 may support prosthetic valve leaflets 20 that operate in lieu of the native valve leaflets. The frame 14 may include couplers 22 for coupling to the deployment apparatus 10, to retain the implant 12 to the deployment apparatus 10 until deployment is desired. The couplers 22 may comprise apertures as shown in FIG. 2, or may have other forms as desired. Although implant 12 is shown in FIG. 2, the use of the deployment apparatus 10 is not limited to the embodiment of implant 12 shown in FIG. 2, and may extend to other forms of implants as desired.

Referring back to FIG. 1, the deployment apparatus 10 may include an elongate shaft 24 including a proximal end 26 and a distal end 28. A housing in the form of a handle 30 may be positioned at the proximal end 26 of the elongate shaft 24. The handle 30 may be configured for an individual to grip to utilize when operating the deployment apparatus 10. The elongate shaft 24 may extend outward from the handle 30 and may be configured to be inserted into a subject to be directed to a desired treatment site of the subject. The elongate shaft 24 may be configured to be inserted into the vasculature of the subject, or otherwise may be inserted into the vasculature of the subject. Such insertion may be percutaneous and minimally invasive, such as transfemoral entry. Other forms of entry, such as transapical may be utilized as well. The handle 30 remains exterior to the subject during insertion.

The elongate shaft 24 of the deployment apparatus 10 may include a deployment device that it utilizes in the deployment procedure. The deployment device may comprise any device utilized in the deployment procedure. The deployment device, for example, may comprise an implant retention device 32, which for example may comprise a sheath to forming a capsule over an implant retention area. The implant may be retained in the implant retention device 32 until the desired time for deployment of the implant. The elongate shaft 24 may be inserted into the subject and navigated to the desired deployment location to position the implant retention device 32 as desired. The deployment apparatus 10 may then be operated to deploy the implant from the implant retention device 32 by the implant retention device 32 retracting the sheath to expose the implant, allowing the implant to be deployed. In embodiments, the deployment device may comprise a dilation device. For example, the deployment device may comprise an expandable balloon that is utilized to dilate a native valve prior to implantation of the implant, or during or following implantation. In embodiments, the deployment device may comprise a combination of a dilation device and an implant retention device, or other devices. For example, a single deployment apparatus may be configured to perform the deployment operation of dilation, as well as the deployment operation of implant deployment. Other methods may be performed by the deployment apparatus as well.

The elongate shaft 24 may further include a nose cone 34 at the distal end 28 of the elongate shaft 24. The nose cone 34 may form the tip of the elongate shaft 24 and may be pliable to avoid injury to portions of the subject contacted by the tip of the elongate shaft 24.

Although features of the disclosure are disclosed in regard to the deployment apparatus 10 shown in FIG. 1, various other forms of deployment apparatuses may be utilized with the embodiments disclosed herein.

A possible issue surrounding insertion and navigation of a deployment apparatus 10 to an implant location, as well as actual deployment of an implant to an implant location, is the possibility of particles entering into the bloodstream of the subject or otherwise being produced. Such particles may comprise emboli or other forms of particles that may have severe deleterious effects for a subject. For example, such particles may produce strokes among other maladies of the subject. Accordingly, it may be advantageous to provide a filter for trapping such particles.

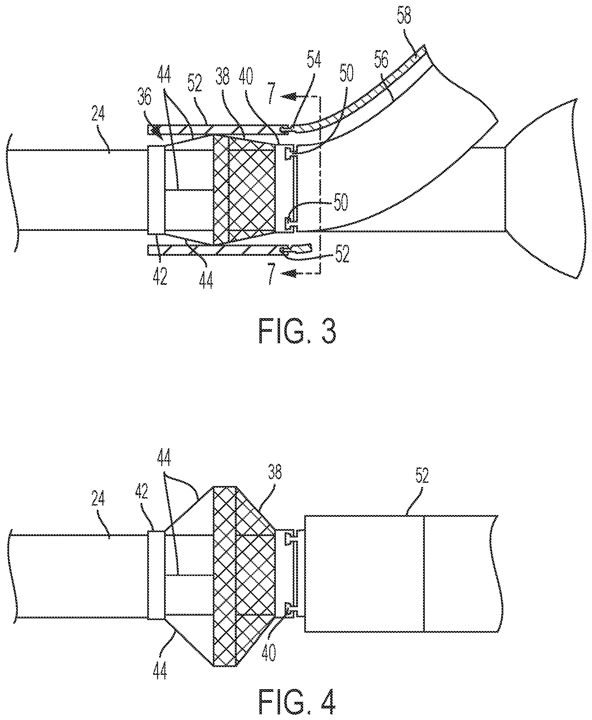

FIG. 3 illustrates a side schematic view of a filter 36 that may be utilized to trap particles in the filter 36. Such particles may include emboli or other forms of particles. The filter 36 may be configured to allow blood to pass therethrough and trap emboli or other forms of particles. The filter 36 may include a filter body 38, a filter base 40, a filter support 42, and one or more support tethers 44. The filter 36, together with a deployment apparatus 10 as shown in FIG. 1 for example, may form a deployment system for an implant, such as the implant 12 discussed in regard to FIG. 2. The filter 36 may be configured to extend radially outward from the elongate shaft 24 of the deployment apparatus 10 in a deployed state.

The filter body 38 may comprise a flattened body (which may include a sheet or panel of material) that includes a plurality of openings (as shown in FIG. 3) and/or may be formed into a shape (such as conical or another shape). The openings may be formed in variety of manners. For example, the filter body 38 in one embodiment may be made of a woven material or may otherwise be arranged in a grid that leaves openings between the body of the material. The filter body 38 may comprise a mesh material, forming a grid that leaves openings between the body of the material. In embodiments, the filter body 38 may be made of a unitary sheet of material that has micropores produced on the material. In embodiments, the filter body 38 may comprise a diffusive material that allows liquid diffusion through the material yet captures particles. Other forms of filter bodies 38 may be utilized as desired.

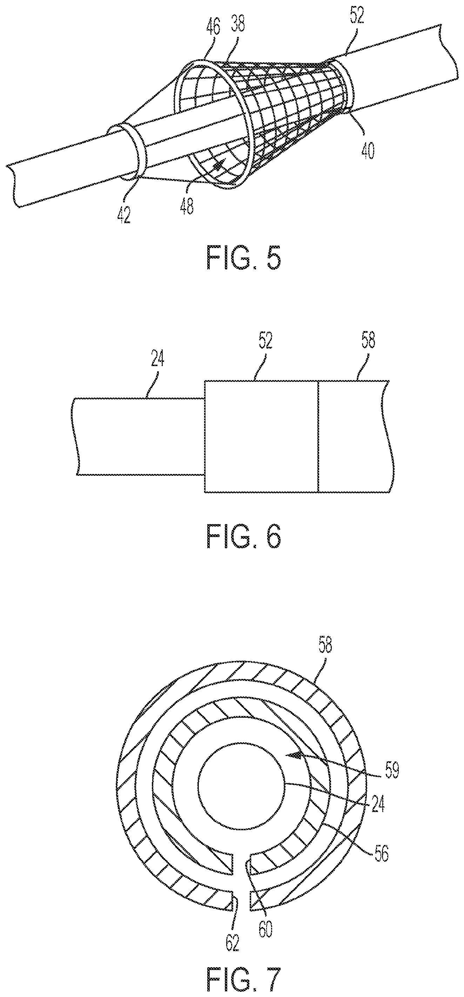

The filter body 38 may comprise a flexible movable body that may be formed into shapes and may be configured to transition from an undeployed state or configuration to a deployed state or configuration. FIG. 3, for example, illustrates the filter body 38 in an undeployed, compressed, or flattened state in which the outer radius of the filter body 38 is less than in the deployed, uncompressed, or expanded state. An example of the filter body 38 in a deployed, uncompressed, and expanded configuration is shown in FIG. 4 in which the filter body 38 has a larger outer radius than in the unexpanded configuration. The filter 36 may extend radially outward from the elongate shaft 24 to a greater radial extent than in the undeployed state. Further, as shown in FIG. 5, the filter body 38 may form an opening 46 of an interior cavity 48 for retaining the particles and that is configured to trap the particles. The filter body 38 may extend around the interior cavity 48. In embodiments, the opening 46 is positioned at the distal portion of the filter body 38.

Referring back to FIG. 3, the filter body 38 may extend around the elongate shaft 24 and particularly around the outer surface of the elongate shaft 24. The filter body 38 may extend around the entire outer circumference of the elongate shaft 24.

The filter body 38 may be configured to slide along the outer surface relative to the elongate shaft 24 in either the undeployed or deployed configuration. The filter body 38 may be configured to slide in proximal and distal directions.

The filter base 40 may be positioned at a proximal portion of the filter body 38. A proximal portion of the filter body 38 may be configured to couple to the filter base 40. The filter base 40 may couple the filter body 38 to the elongate shaft 24 and may comprise a ring or other body. The filter base 40 may extend around the entire outer circumference of the elongate shaft 24 similar to the filter body 38. The filter base 40 may include couplers 50 configured to couple to a control device such as a sheath for controlling the position of the filter body 38. The couplers 50 may comprise ball and socket couplers as shown in FIG. 3, or may comprise any other form of coupler. The filter base 40 may be configured to slide along the outer surface of the elongate shaft 24.

The filter support 42 may include a body that couples to and supports a distal portion of the filter body 38. The filter support 42 may comprise a ring or other body and may be configured to extend around the elongate shaft 24. The filter support 42 may be configured to be positioned distal of a proximal portion of the filter body 38 and configured to couple to the elongate shaft 24. The filter support 42 may be configured to couple to tethers 44 that couple the filter support 42 to the distal portion of the filter body. The filter support 42 may be configured to slide along the outer surface of the elongate shaft 24 and slide relative to the filter body 38.

The support tethers 44 may be configured to couple to a distal portion of the filter body 38, such as the portion proximate to the opening 46. The support tethers 44 may extend from the filter support 42 to the distal portion of the filter body 38. The support tethers 44 may comprise cords or wires or other forms of tethers as desired. The tethers 44 may be configured to extend radially outward from the outer surface of the elongate shaft 24 when the filter body 38 transitions to the deployed state.

A retainer may be utilized to retain the filter 36 in an undeployed state or configuration until the time to deploy the filter 36. As shown in FIG. 3, the retainer may comprise a sheath 52 configured to extend over the filter 36, particularly the filter body 38. However, in other embodiments, the retainer may have a different form, including a clip, a latch, one or more wires, or another form of retainer. The sheath 52 may extend over the filter 36, and in some embodiments over the entirety of the filter 36, and may have a diameter sized to press against the filter 36 to maintain the filter 36 in the undeployed configuration. The sheath 52 may be configured to slide along the outer surface of the elongate shaft 24, in a proximal direction, to allow the filter 36 to transition to the deployed state. The sheath 52 is shown in cross section in FIG. 3. The sheath 52 may include couplers 54 that may be configured similarly as the couplers 50 of the filter base 40.

A portion or all of the filter 36 may be self-expanding. The filter 36 may be made of a shape memory material to automatically transition to a deployed configuration upon being released by the retainer shown in the form of the sheath 52. Such a shape memory material may comprise nitinol or another form of shape memory material. For example, all or a portion of the filter body 38 may be made of a shape memory material to cause the filter body 38 to transition to the deployed configuration upon the sheath 52 uncovering or otherwise exposing the filter body 38. In embodiments, the tethers 44 may be made of a shape memory material to extend the filter body 38 outward into a deployed configuration.

The filter 36 may be coupled to a control device for controlling operation of the filter 36. The control device may control the expansion of the filter 36 and may control the position of the filter 36 upon the elongate shaft 24. As shown in FIG. 3, the control device may comprise two sheaths 56, 58. The sheath 58 may extend over the sheath 56. The inner sheath 56 may couple to the filter 36 (particularly the coupler 50 of the filter base 40) and the outer sheath 58 may couple to the retainer in the form of the sheath 52. The sheaths 56, 58 may both be configured to have a distal end coupled to the respective filter 36 and sheath 52, and may include a body that slides along the elongate shaft 24 to vary a position of the filter 36 and the sheath 52 upon the elongate shaft 24. Thus, as the sheaths 56, 58 are slid distally along the elongate shaft 24 the filter 36 and sheath 52 are slid distally, and as the sheaths 56, 58 are slid proximally along the elongate shaft 24 the filter 36 and sheath 52 are slid proximally.

The control device in the form of the sheaths 56, 58 may be utilized with the elongate shaft 24 without significant modification of the elongate shaft 24 or the handle 30. For example, referring to FIG. 7, which illustrates a cross sectional view along line 7-7 in FIG. 3, each sheath 56, 58 may include a respective longitudinal cut or gap 60, 62 extending longitudinally along the sheath 56, 58 such that each sheath 56, 58 may be passed over the elongate shaft 24 through the gap openings. Thus, referring back to FIG. 3, each sheath 56, 58 may slid onto the elongate shaft 24 through the gaps 60, 62 shown in FIG. 7. The sheaths 56, 58 accordingly may be made flexible to allow for the elongate shaft to pass through the gaps 60, 62.

The elongate sheath 56 may be coupled to the filter 36 and may have a length with the longitudinal cut or gap 60 extending along the length of the elongate sheath 56. An interior cavity 59 may be configured to receive the elongate shaft 24 of the deployment apparatus. The elongate sheath 56 may be configured to couple to the elongate shaft 24 of the deployment apparatus by the elongate shaft 24 being passed through the longitudinal cut or gap 60 of the elongate sheath 56 to be positioned within the interior cavity 59 of the elongate sheath 56. The elongate sheath 56 is configured to be slid onto the elongate shaft 24 with the elongate shaft 24 passing through the cut 60 of the elongate sheath 56. The elongate sheath 56 surrounds the elongate shaft 24 as shown in FIG. 7. The elongate sheath 58 joins with the elongate shaft 24 in a similar manner through the cut 62, and extends over the elongate sheath 56. Distal portions of the sheaths 56, 58 may be engaged with the elongate shaft 24 and proximal portions of the sheath 56, 58 may be disengaged, with the proportion of the engaged and disengaged portions varying due to the movement of the sheaths 56, 58 either on or off the elongate shaft 24.

In this manner, the distal movement of the filter 36 and the sheath 52 may be controlled by the length of the sheath 56, 58 that is slid onto the elongate shaft 24 distally, and the proximal movement of the filter 36 and the sheath 56, 58 may be controlled by the length of the sheath 56, 58 that is slid off of the elongate shaft 24 proximally in a reverse operation. The elongate shaft 24 may be inserted into the interior cavity 59 through the cuts 60, 62 extending along the lengths of the elongate sheaths 56, 58. A distal portion of the elongate sheath 56 may be coupled to the filter 36. The elongate sheaths 56, 58 may be slid distally along the elongate shaft 24 according to embodiments herein, and the filter 36 may be deployed within the vasculature of the subject.

Further, rotational control of the rotational orientation of the filter 36 and sheath 52 may be provided by rotating the proximal portion of the sheath 56, 58 that the individual is gripping.

Other control devices may be utilized, including wire guided control or motorized control, among other forms of control devices. The housing comprising the handle 30 may be modified in certain embodiments to allow a sheath to pass through the handle 30.

In operation, the filter 36, covered with the sheath 52 may be advanced distally along the elongate shaft 24 to a desired position. The sheaths 56, 58 shown in FIG. 3 may be slid onto the elongate shaft 24 to increase the length of the sheaths 56, 58 upon the elongate shaft 24 and move the filter 36 and sheath 52 proximally. In certain embodiments, the filter 36 may be slid into the subject along with the elongate shaft 24, and thus may remain in position with the elongate shaft 24 during insertion of the elongate shaft 24. The filter 36 may subsequently have its position adjusted to a desired location.

The filter 36 may be deployed by the sheath 52 being withdrawn proximally to uncover the filter 36. Such a configuration is shown in FIG. 4. The filter 36 transitions to the expanded or deployed state or configuration, with an increased outer radius than shown in FIG. 3. The tethers 44 may restrain the distal portion of the filter 36 from further expansion. The filter 36 may form a configuration as shown in perspective view in FIG. 5. The filter body 38 may form a conical shape enclosing an interior cavity 48. The conical shape may increase in size from a proximal portion of the filter body 38 to a distal portion of the filter body 38. The filter body 38 may transition to the deployed state in which the filter body 38 extends radially outward from the elongate shaft 24 and increases in size from the proximal portion to the distal portion. The filter body 38 is configured to trap particles in the filter body 38.

The proximal end of the filter body 38 couples to the filter base to prevent particles from passing through the filter body 38 at the apex of the conical shape. In other embodiments, other shapes of filter bodies 38 may be utilized, including spheroid or dome shaped, cylindrical, rectangular, or triangular, among others. A conical shape may preferably have a large symmetrical opening 46 at a distal portion of the filter body 38 that may contour to the shape of the vasculature of the subject. The distal portion may be made flexible and may comprise a contact surface for contacting an interior of the vasculature. The taper of the conical shape may drive the trapped particles to a central position at the proximal end of the filter body 38.

The filter 36 may be deployed to allow fluid, including colloid fluid (such as blood), to pass through, yet to trap particles. As such, the openings of the filter 36 may be sized as desired to prevent particles of a certain size from passing through and may yet allow colloidal members of fluid to pass.

With the desired particles trapped by the filter 36, the sheath 52 may be advanced distally by the sheath 58 being advanced distally. Such a movement may slide the sheath 52 back over the filter 36 to produce a configuration as shown in FIG. 6. The filter body 38 may close upon any particles trapped therein, to retain the particles under the sheath 52 and within the filter body 38. The entire elongate shaft 24 may then be retracted along with the filter 36 and sheath 52, or the filter 36 and sheath 52 may be retracted separately.

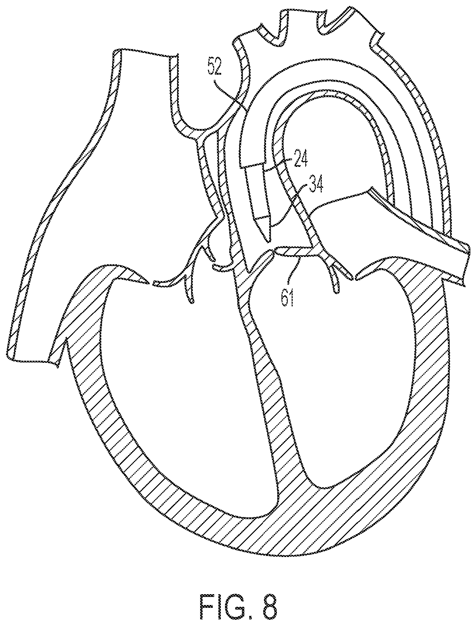

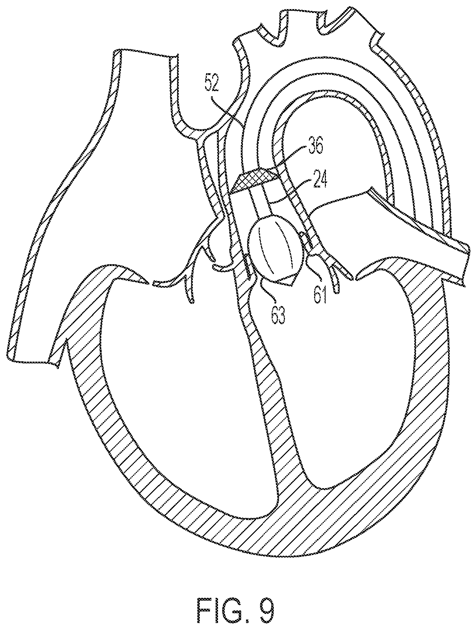

FIGS. 8 and 9 illustrate a possible position of the filter 36 in use. The filter 36 inserted within the subject, with the filter 36 positioned upon an elongate shaft 24 of a deployment apparatus. The filter 36 is deployed within vasculature of the subject, with the filter 36 extending radially outward from the elongate shaft 24 and configured to trap particles in the filter 36. The elongate shaft 24 may be advanced distally to the desired implantation location for the implant as shown in FIG. 8 as the aortic valve 61. The filter 36 may be slid upon the elongate shaft 24 if desired. The filter 36 may be positioned proximal of the deployment device of the deployment apparatus. Prior to, during, or possibly even after the deployment of the implant occurs, the filter 36 may be placed in position.

As shown in FIG. 9, the filter 36 is positioned proximal of the distal end of the elongate shaft 24 and proximal of the deployment device 63 and is configured to trap particles passing proximally from the distal end of the elongate shaft 24 and from the deployment device 63.

The filter 36 is shown to be deployed within the aortic arch proximal of the aortic valve 61. The filter 36 may be deployed via operation of a control device (e.g., the sheaths 56, 58) that may release the retainer and cause the filter 36 to transition from an undeployed state to a deployed state. The filter 36 is positioned within a blood vessel of the subject and is positioned proximal of a heart valve (the aortic valve) that the deployment apparatus is deploying a device to. The filter 36 is positioned distal of the arteries extending from the aortic arch, to trap any particles that otherwise may pass through such arteries. The filter 36 is positioned proximal of a deployment device of the deployment apparatus. The opening of the filter 36 faces distally and the interior cavity of the filter 36 is configured to retain particles travelling proximally from the deployment device of the deployment apparatus.

The deployment device 63 as shown in FIG. 9 may comprise an inflatable balloon, which may dilate the aortic valve 61 prior to deployment of the implant. The filter 36 may be slid along the elongate shaft 24 to be placed in position. Here, the filter 36 may beneficially trap any calcification or other particles that are released in the proximal direction during an aortic valve deployment procedure. The filter 36 may be utilized to trap particles as a result of an implant deployment procedure, which as shown in FIG. 9 may include inflating a balloon to dilate the aortic valve 61. Such other procedures may include actual implantation of the implant, and any subsequent dilation of the implant or the aortic valve, among other procedures. The filter 36 may then be transitioned to the undeployed state and retracted along with the elongate shaft 24 and withdrawn from the subject. The filter 36, when closed, may trap the particles therein for removal from the subject.

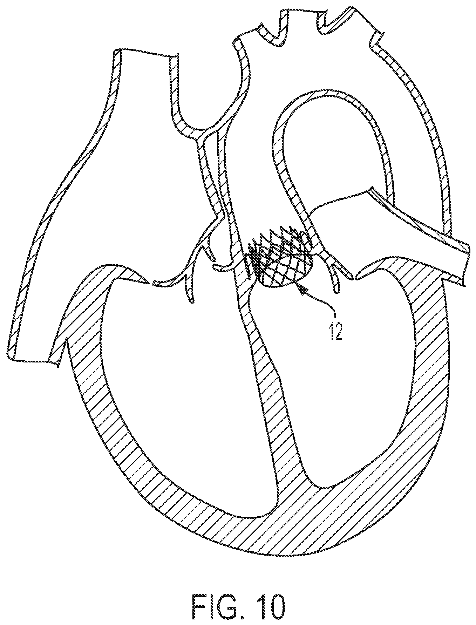

FIG. 10 illustrates an implant 12 that may be deployed by the deployment apparatus and may remain in position within the native valve. The deployment apparatus and filter 36 may be retracted proximally and withdrawn from the subject.

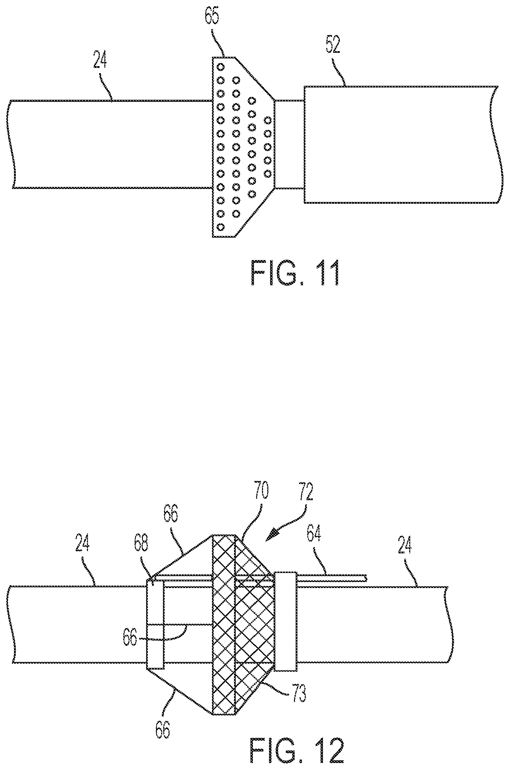

FIG. 11 illustrates an embodiment of a filter 65 that may include a plurality of micropores to allow colloidal fluid to pass through, yet allow the filter body to trap particles. The filter 65 may not utilize a filter base or filter support or tethers. The filter 65, for example, may be coupled directly to a position on the elongate shaft 24. A control device in the form of a sheath 52 may be utilized to expand and collapse the filter 65 in a manner disclosed herein.

FIG. 12 illustrates an embodiment of the presently claimed invention in which a control device may be in the form of a control tether 64 such as a push or pull tether. In embodiments, other forms of control devices may be utilized.

The control device passes through a proximal portion 72 of the filter body 73 and couples to the filter support 68. The control device is configured to be slid relative to the filter body 73 to slide the filter support 68 to move the support tethers 66 and transition the filter body 73 to the deployed state. The control device is configured to be slid proximally relative to the filter body 73 to slide the filter support 68 to move one or more support tethers 66 and transition the filter body 73 to the deployed state.

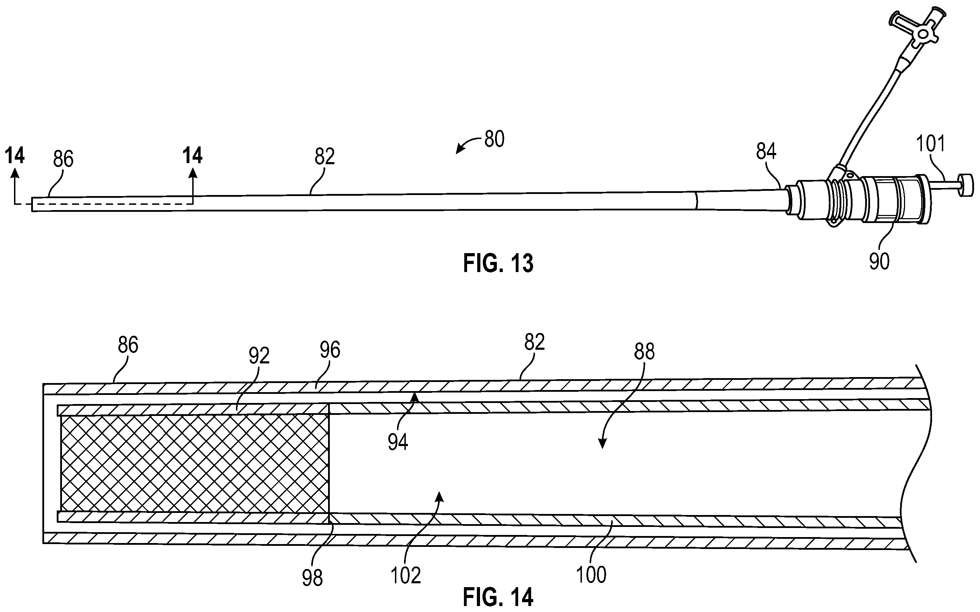

The control device may be slid to move the tethers 66 and accordingly expand or collapse the filter 70 as desired. The control device may have a proximal end configured to be controlled by an individual, similar to the proximal end 101 shown in FIG. 13 for example. The control device may be slid to transition the filter body 73 to the deployed state. The filter 70 may remain in position on the elongate shaft 24 or may have a variable position controlled by another control device if desired (e.g., the filter 70 may be slidable along the elongate shaft 24). The control device may be configured to be slid distally relative to the filter body 73 to slide the filter support 68 to move one or more support tethers 66 and transition the filter body 73 to an undeployed state. Further, by varying the position of the control device, by sliding the control device, a size of the distal portion of the filter body 73 may be controlled.

Various other modifications are within the scope of this disclosure.

FIG. 13 illustrates an embodiment of a catheter sheath that may be utilized in a catheter system. As shown, the catheter sheath comprises an introducer sheath 80, yet other forms of catheter sheaths may be utilized herein. The catheter sheath that may utilize a filter. The introducer sheath 80 may include an elongate shaft 82 having a proximal end 84 and a distal end 86 and an interior lumen 88 (marked in FIG. 14) configured for another apparatus to pass through. For example, an apparatus such as a deployment apparatus 10 as shown in FIG. 1 may be utilized in embodiments.

A handle 90 may be positioned at the proximal end 84 of the elongate shaft 82 and may be configured to be gripped by a user. The introducer sheath 80 may be utilized to be introduced into a subject and may serve as a channel for other apparatuses to pass through to perform procedures within a subject.

In embodiments, the introducer sheath 80 may be flexible and configured to flex along with a curved or tortuous path of the vasculature. In embodiments, the introducer sheath 80 may be sufficiently long to pass, for example, from an entry point in the subject's leg, over the aortic arch and may have the distal end 86 positioned proximate the native aortic valve. Other lengths and configurations of introducer sheaths 80 may be utilized in embodiments.

FIG. 14 illustrates a cross sectional view of the distal end 86 of the introducer sheath 80. A filter 92 may be positioned within the interior lumen 88 of the introducer sheath 80 and may be retained within the interior lumen 88 in an undeployed or unexpanded state. As such, the filter 92 may be retained within the lumen 88 by the interior surface 94 of the wall 96 of the elongate shaft 82.

The filter 92 may be configured to be biased to expand radially outward from the interior lumen 88. The filter 92, for example, may have a shape memory that causes the filter 92 to expand radially outward upon being passed out of the lumen 88 of the elongate shaft 82. The filter 92 may otherwise be configured to expand, for example, via a spring bias force or other device to cause the filter 92 to expand radially outward.

The filter 92, in embodiments, may comprise a mesh material or other material configured to trap particles, yet allow fluid, including colloidal fluid such as blood, to pass through. Other configurations of filters, such as configurations utilizing micropores or other configurations as disclosed herein, may be utilized.

The filter 92 may have a proximal portion 98 that is coupled to a control device 100. The control device 100, for example, may comprise a shaft that extends along the lumen 88 of the elongate shaft 82. The shaft may comprise a sheath having its own interior lumen 102 that may allow apparatuses to pass therethrough. The shaft may have a proximal end 101 (as shown in FIG. 13) that a user may control to control movement of the filter 92. In embodiments, other configurations of control devices such as tethers or other forms of devices for controlling movement of the filter 92 may be utilized.

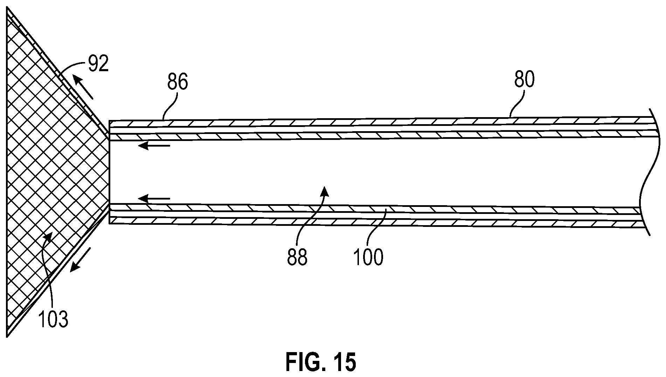

FIG. 15 illustrates the filter 92 expanded out of the distal end 86 of the introducer sheath 80. The control device 100 may be advanced distally by the user distally advancing the proximal end 101 (shown in FIG. 13) of the control device 100. The filter 92 may advance distally and may expand radially outward as shown in FIG. 15. The shape may be a conical shape as shown in FIG. 15, or another shape as desired. The filter 92 may include an interior cavity 103 configured to trap particles therein. The filter 92, in the expanded state shown in FIG. 15, may be positioned to allow an apparatus to pass through the interior lumen 102 of the control device 100 and the interior lumen 88 of the introducer sheath 80.

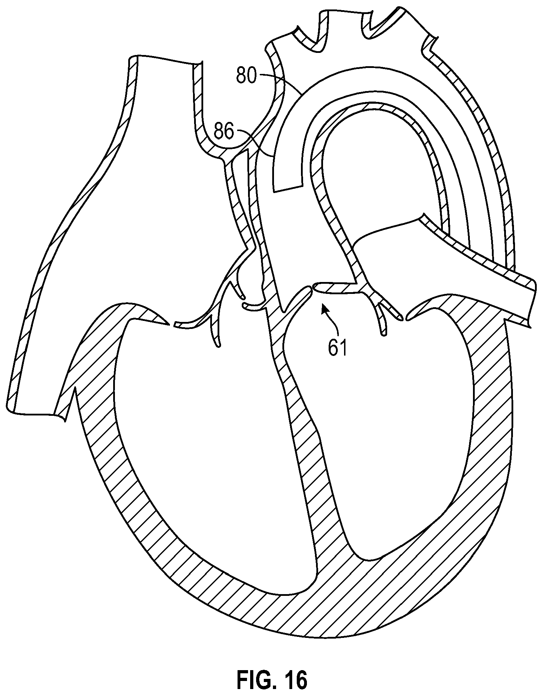

FIGS. 16-18, for example, illustrate an exemplary use of the introducer sheath 80. The introducer sheath 80 may have a length sufficient to extend over the aortic arch and towards the aortic valve 61 as shown in FIG. 16. The introducer sheath 80 may have sufficient flexibility to extend over the aortic arch.

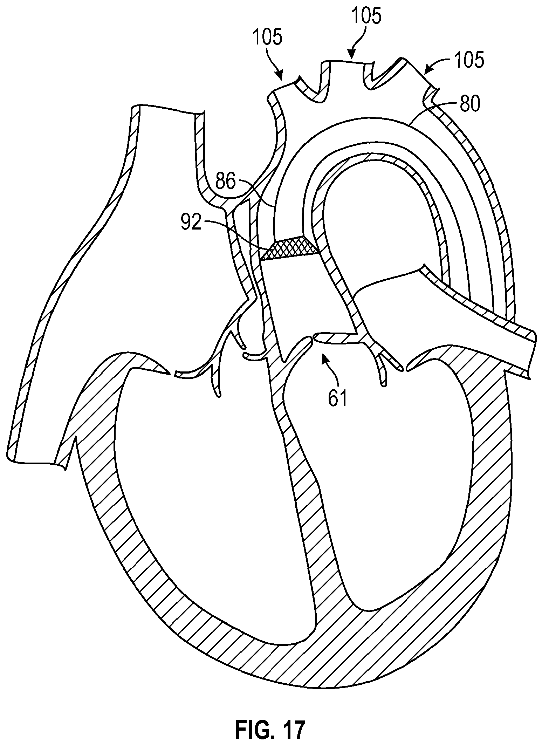

The filter 92 may then be extended out of the distal end 86 of the introducer sheath 80. The filter 92 may be advanced distally as shown in FIG. 15 to be deployed at the desired position. The filter 92, as shown in FIG. 17, may be deployed at a position that is proximate the aortic valve 61 and between the aortic valve 61 and channels that particle may flow into, such as the ostia 105 positioned along the aortic arch. As such, the filter 92 may reduce the possibility of particles from entering such ostia 105 and producing an adverse effect for the subject.

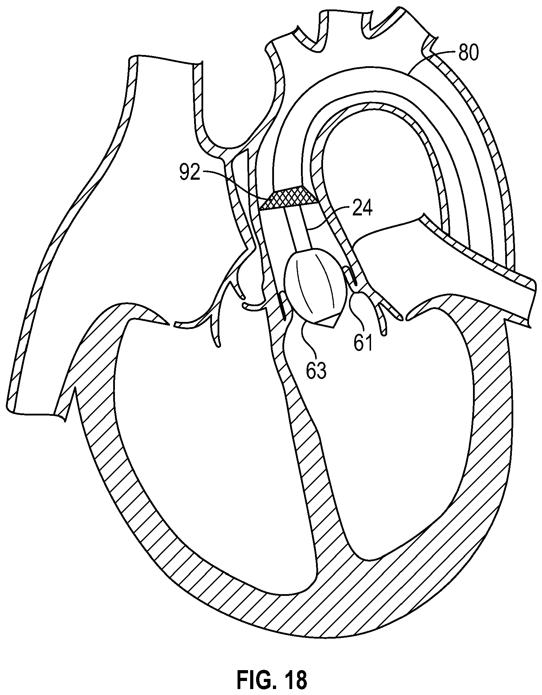

FIG. 18, for example, illustrates an elongate shaft 24 of a deployment apparatus passing through the introducer sheath 80, for deployment to the aortic valve 61. Various procedures may be provided at the aortic valve 61. Such procedures may release particles, which may flow in a downstream direction and may be captured by the filter 92.

Following a procedure being performed to the aortic valve 61, the deployment apparatus may be retracted from the introducer sheath 80 and the filter 92 and introducer sheath 80 may be retracted as well. The particles trapped within the filter 92 may be removed with the filter 92 and introducer sheath 80. In embodiments, the control device 100 shown in FIG. 15 may be retracted to cause the filter 92 to retract into the interior lumen 88 of the introducer sheath 80.

Various modifications to the filters and methods of utilization may be provided.

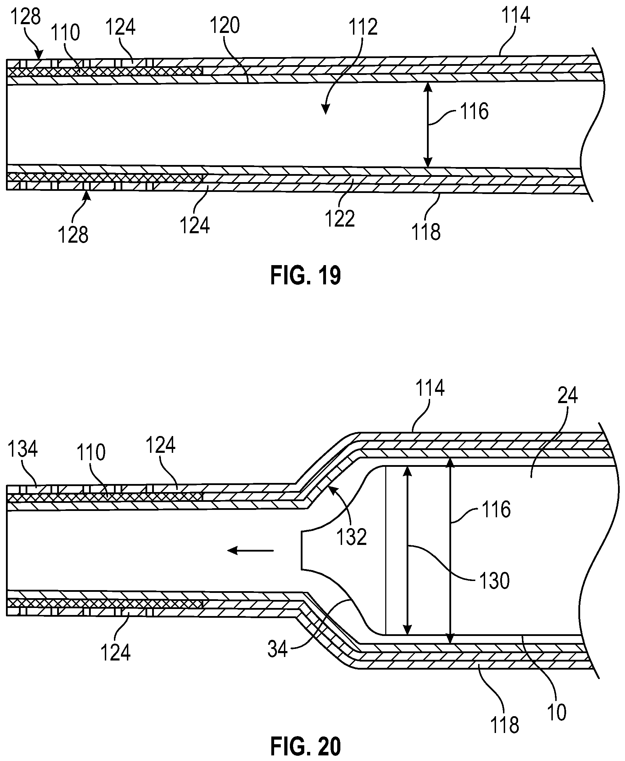

FIG. 19, for example, illustrates an embodiment of a filter 110 configured to automatically deploy upon an apparatus being passed through the interior lumen 112 of a catheter sheath. In embodiments, the catheter sheath may comprise an expandable catheter sheath that is configured to be inserted into a vasculature and have an interior lumen 112 configured for an apparatus to be passed through, the interior lumen 112 having an interior diameter 116 that is configured to increase upon the apparatus passing through the interior lumen 112. The catheter sheath may comprise an expandable introducer sheath 114 that is configured to expand radially outward.

The interior lumen 112 of the expandable introducer sheath 114 may have a diameter 116. The diameter 116 may be configured to increase upon an apparatus passing through the interior lumen 112. The wall 118 of the expandable introducer sheath 114, for example, may be configured to expand. The expansion may occur due to the wall 118 deforming upon the apparatus passing through the interior lumen 112. The wall 118, for example, may be constructed of an expandable or tearable material that deforms upon an apparatus passing through the interior lumen 112. The deformation may comprise a plastic deformation in embodiments that may reduce the possibility of the wall 118 returning to the narrow diameter 116 shown in FIG. 19. In embodiments, the wall 118 may include one or more folded portions that may allow the wall 118 to expand upon an apparatus passing through the interior lumen.

The filter 110 may be positioned at a distal portion of the expandable catheter sheath and may be configured to expanded from an undeployed state radially outward to a deployed state in response to the apparatus passing through the interior lumen 112 and applying a radially outward force to the interior lumen 112.

The filter 110 may be coupled to the introducer sheath 114 and may be coupled to the wall 118. The filter 110, in embodiments, may be embedded within the wall 118, and may be embedded in an unexpanded state as shown in FIG. 19. The wall 118, for example, may comprise a multi-layered structure, and one of the layers may include the filter 110 in an unexpanded state.

Layers may include a liner layer 120 that may be positioned radially inward of the filter 110 and between the filter 110 and the interior lumen 112. The liner layer 120 may be configured to contact the apparatus as the apparatus passes through the interior lumen 112 and applies the radial outward force to the interior lumen. The filter 110 may be positioned in a mid-layer 122 that may extend over the liner layer 120. In embodiments, an outer layer or retention layer 124 may be positioned radially outward of the filter 110 and configured to retain the filter 110 in the undeployed state. The retention layer 124 may extend over the filter 110 to retain the filter 110 in the undeployed or unexpanded state. In embodiments, the retention layer 124 may include pores 128 or other forms of openings that may be configured to allow fluid to flow therethrough when the filter 110 is deployed.

The filter 110 in the unexpanded state may have a cylindrical shape, or may have another shape as desired.

An apparatus, such as an elongate shaft 24 of a deployment apparatus 10, may be passed through the interior lumen 112 and may have a diameter 130 that is larger than the diameter 116 of the interior lumen 112 shown in FIG. 19. As shown in FIG. 20, the relatively large diameter 130 of the apparatus, as the apparatus is passed through the interior lumen 112, may expand the wall 118 of the introducer sheath 114 radially outward. A radially outward force may be applied by the apparatus to the interior surface 132 of the wall 118. The radially outward force may deform the wall 118, as discussed herein.

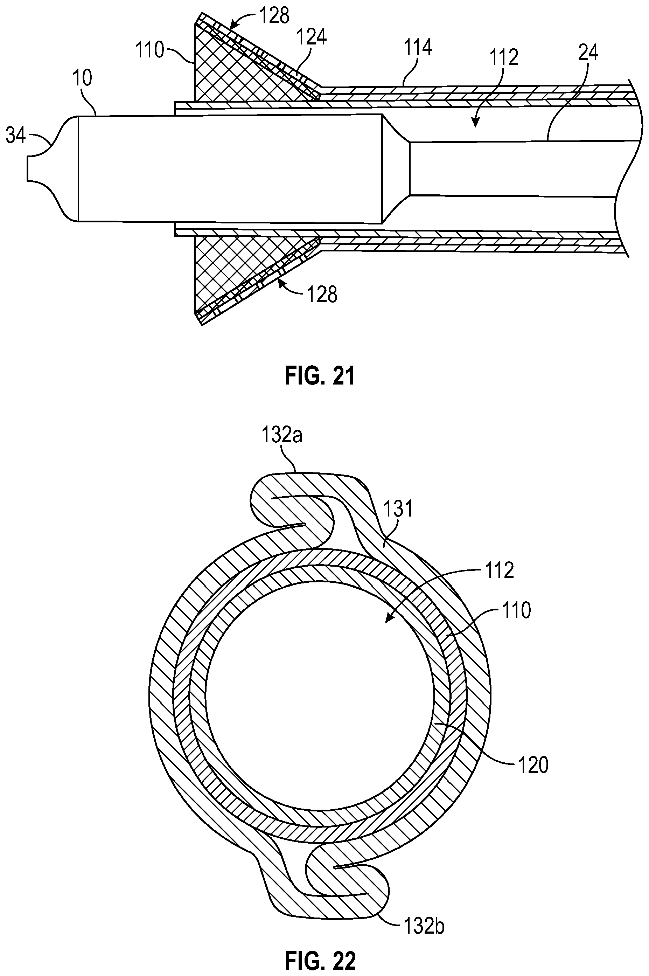

As the apparatus approaches the filter 110 of the introducer sheath 114, which may be positioned at the distal end 134 of the introducer sheath 114, the force of the apparatus against the wall 118 may deform the retention layer 124 and may reduce the force applied by the retention layer 124 against the filter 110. The retention layer 124, for example, may be configured to deform in the response to the apparatus passing through the interior lumen 112 and applying the radial outward force to the interior lumen 112. The retention layer 124 may be deformed to change its structural configuration such that the retention force applied by the retention layer 124 to the filter 110 may be reduced. Such reduction may be due to a deformation of the retention layer 124, which may be a plastic deformation of the retention layer 124, a tearing of the retention layer 124, or an unfolding of at least one fold of the retainer layer 124 in an embodiment in which the retention layer 124 includes at least one fold (such as an embodiment as shown in FIG. 22). In an embodiment in which the retention layer is torn, a tear or split line, such as perforations, may be provided on the retention layer 124. The retention layer 124 may reduce its retention force against the filter 110, and allow the filter 110 to expand radially outward to the configuration shown in FIG. 21, for example.

As such, as an apparatus is passed through the interior lumen of the expandable catheter sheath positioned within the subject, a radially outward force may be applied to the interior lumen 112 to expand the filter 110 positioned at the distal end of the expandable catheter sheath from the undeployed state radially outward to the deployed state.

The filter 110 may be biased radially outward, and with the reduce retention force applied by the retention layer 124 may be configured to form a shape extending radially outward from the introducer sheath 114, as shown in FIG. 21. The filter 110, as such, may be in position to trap particles therein, in a similar manner as discussed in regard to FIGS. 16-18 for example. The filter 110 may include a filter body that includes a plurality of openings, as disclosed herein. The filter 110 may have a conical shape as disclosed herein when the filter is in the deployed state, with the conical shape increasing in size from a proximal portion of the filter body to a distal portion of the filter body. The retention layer 124 may include pores 128, in embodiments, which may allow the filter 110 to continue to allow fluid, including colloidal fluid such as blood to pass through, yet while retaining particles therein. The filter 110 may filter blood within the subject.

The filter 110 may automatically deploy to the deployed or expanded state shown in FIG. 21 upon an apparatus passing through the interior lumen 112. As such, the activation of the filter 110 may be a passive activation according to embodiments herein.

Upon completion of a medical procedure utilizing the apparatus, the apparatus and introducer sheath 114 may be withdrawn, with the filtered particles retained within the filter 110.

Various configurations of the filters and sheaths disclosed herein may be provided.

FIG. 22, for example, illustrates an embodiment of an introducer sheath in which the retainer layer 131 includes fold portions 132a, b configured to unfold as an apparatus is passed through the interior lumen 112. The unfolding of the fold portions 132a, 132b, may allow the filter 110 to expand to the expanded configuration, as shown in FIG. 21 for example. The introducer sheath may include at least one fold portion in embodiments herein.

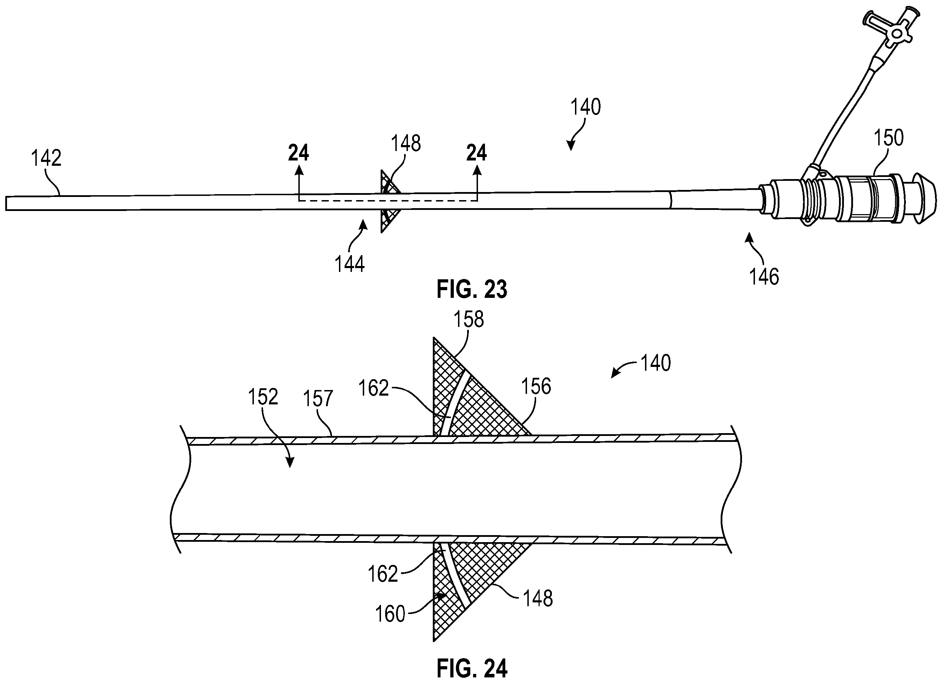

FIG. 23 illustrates an embodiment of a catheter sheath in the form of an introducer sheath 140 configured to be inserted into a vasculature and having a length and an interior lumen configured for an apparatus to be passed through. The introducer sheath 140 may have a distal portion 142, a central portion 144, and a proximal portion 146, with a filter 148 positioned at the central portion 144 of the introducer sheath 140. The introducer sheath 140 may otherwise be configured similarly as the other introducer sheaths disclosed herein. For example, the introducer sheath 140 may be configured to expand in embodiments. The introducer sheath 140 may include a handle 150 coupled to the proximal portion 146 of the introducer sheath 140.

FIG. 24 illustrates a close-up cross-sectional view of the filter 148 positioned on the introducer sheath 140. The introducer sheath 140 may include an interior lumen 152 and a wall 154 extending around the interior lumen 152. The filter 148 may be configured to extend radially outward from the wall 154, and may surround the introducer sheath 140. The filter 148 may have a proximal portion 156 that is coupled to the wall 154 and may have a distal portion 158 that extends in a distal direction relative to the introducer sheath 140. The filter 148 may increase in size from the proximal portion of the filter 148 to the distal portion of the filter 148 in a direction towards a distal portion of the introducer sheath 140. The distal portion 158 may form an opening that leads to an interior cavity 160 for retaining particles therein, similar to the configuration of other filters disclosed herein. In embodiments, the filter 148 may have a conical shape, or another shape as desired when in a deployed state. The filter 148 may include a filter body having a plurality of openings, as disclosed herein. In embodiments, biasing members such as supports 162 may be configured to apply a radially outward force against the filter 148 to bias the filter 148 radially outward to the deployed state. The supports 162, for example, in embodiments, may comprise one or more springs such as leaf springs or other forms of springs. In embodiments, a shape memory for the filter 148 may be provided, utilizing shape memory materials. Any embodiment of filter disclosed herein may utilize a shape memory and shape memory materials as desired.

The filter 148 accordingly may be configured to transition from an undeployed state or state in which the filter 148 is compressed to the introducer sheath 140, and a deployed state as shown in FIG. 24, in which the filter 148 extends radially outward from the introducer sheath 140 to trap particles in the filter 148. The supports 162 may transition the filter 148 towards the deployed state.

The filter 148 is preferably positioned at the central portion 144 or the proximal portion 146 of the introducer sheath 140. The filter 148 preferably is positioned at such a location to enhance the likelihood of the filter 148 trapping particles passing along the introducer sheath 140 as the introducer sheath is inserted into the vasculature, with the introducer sheath possibly contacting the interior surface of the vasculature and releasing particles from the interior surface. Such contact may be more likely at a central 144 or proximal portion 146 of the introducer sheath 140, as typically the diameter of the vasculature is lesser at the entry point of the introducer sheath 140 into the vasculature. As such, particles created upon the entry of the introducer sheath 140 into the vasculature may be greater at a central 144 or proximal portion 146 of the introducer sheath 140 and thus the filter 148 may beneficially be provided at such a location. In embodiments, the filter 148 may be positioned proximate the handle 150.

Further, as the introducer sheath 140 is withdrawn, particles may also be created by the contact of the introducer sheath 140 with the interior surface of the vasculature, and a filter 148 positioned at a central 144 or proximal portion 146 may enhance the ability of the filter 148 to trap such particles.

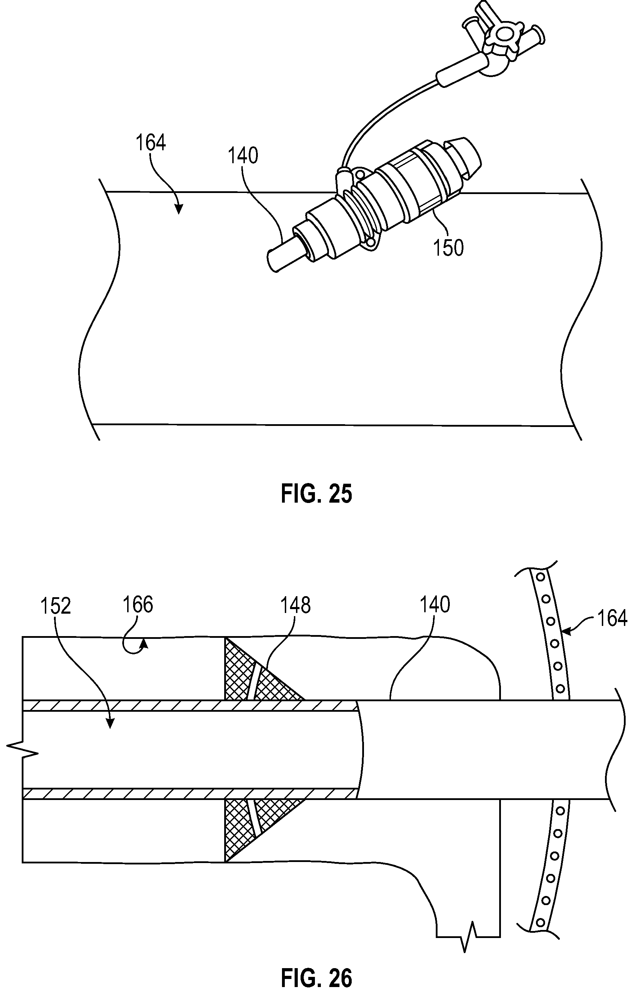

FIG. 25, for example, illustrates the introducer sheath 140 being inserted through a skin 164 and into the vasculature. FIG. 26, for example, illustrates the introducer sheath 140 inserted into a vasculature. The introducer sheath 140 is passed through the skin 164 and positioned within the vasculature, such as a venous structure, which may be an artery or vein. The filter 148 extends radially outward and may contact the interior surface 166 of the vasculature. The handle 150 remains exterior to the skin. Notably, the introducer sheath 140 itself during insertion or withdrawal, may contact the interior surface 166 of the vasculature. Thus, particles may be released from the interior surface 166 and may be trapped by the filter 148.

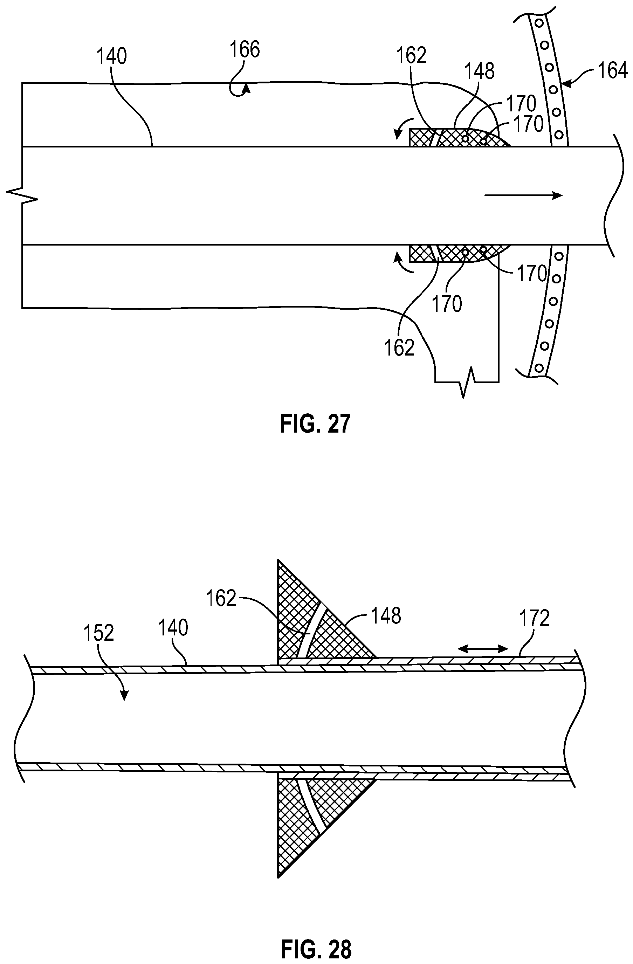

At a desired time, the introducer sheath 140 may be withdrawn from the vasculature. Such a time may be at the completion of a procedure within the subject. FIG. 27, for example, illustrates a withdrawal procedure. During withdrawal, the introducer sheath 140 may contact the interior surface 166 of the vasculature and release further particles 170. Such particles 170 may be trapped by the filter 148 and may be retained between the filter 148 and the exterior surface of the introducer sheath 140. The angle of the filter 148 may be such that as the introducer sheath 140 is withdrawn from the vasculature as shown in FIG. 27, the bias of the support 162 may be overcome and the filter 148 may collapse to transition to an unexpanded or undeployed state. The particles 170 accordingly may be trapped by the filter 148 between the filter 148 and the introducer sheath 140 with the filter 148 in the undeployed state and withdrawn from the subject. Such an operation may occur automatically when the filter 148 is retracted from the vasculature due to the angled shape of the filter 148.

Various other configurations of filters may be utilized in embodiments. FIG. 28, for example, illustrates an embodiment of the filter 148 positioned upon a jacket or sheath 172 that is slidable relative to the introducer sheath 140. As such, the filter 148 may be variably positioned upon the introducer sheath 140. In such an embodiment, as the introducer sheath 140 is withdrawn from the vasculature, the filter 148 may be held in position at the interior surface 166 of the vasculature. As such, the filter 148 may be in position to trap all the particles produced by the introducer sheath 140 contacting the interior surface 166 while being retracted proximally out of the subject. Once the introducer sheath 140 is fully retracted, the jacket or sheath 172 coupled to the filter 148 may be retracted and collapsed by being drawn through the opening in the skin, in a similar manner as shown in FIG. 27.

The configuration of the filters and other components of the deployment systems may be varied in other embodiments.

The use of a filter disclosed herein is not limited to use with a deployment system or a deployment apparatus and may extend to use with any medical device to be inserted or withdrawn within a subject. For example, the use may extend to general medical cannula for insertion into a portion of a subject.

A filter may be utilized in a variety of subjects and procedures. Subjects include (but are not limited to) medical patients, veterinary patients, animal models, cadavers, and simulators of the cardiac and vasculature system (e.g., anthropomorphic phantoms and explant tissue). Procedures include (but are not limited to) medical and training procedures.

The deployment apparatus and the systems disclosed herein may be used in transcatheter aortic valve implantation (TAVI). The deployment apparatus and the systems disclosed herein may be utilized for transarterial access, including transfemoral access, to a subject's heart. In embodiments, various forms of implants may be delivered by a deployment apparatus utilized with system herein, such as stents or filters, or diagnostic devices, among others. The deployment apparatus may be utilized for mitral, tricuspid, and pulmonary replacement and repair as well. Other forms of implants may include stents, clips, and sutures that may be used for valve repair, among other forms of implants.

The deployment systems may be utilized in transcatheter percutaneous procedures, including transarterial procedures, which may be transfemoral or transjugular. Transapical procedures, among others, may also be utilized.

Features of embodiments may be modified, substituted, excluded, or combined.

In addition, the methods herein are not limited to the methods specifically described and may include methods of utilizing the systems and apparatuses disclosed herein.

In closing, it is to be understood that although aspects of the present specification are highlighted by referring to specific embodiments, one skilled in the art will readily appreciate that these disclosed embodiments are only illustrative of the principles of the subject matter disclosed herein. Therefore, it should be understood that the disclosed subject matter is in no way limited to a particular methodology, protocol, and/or reagent, etc., described herein**.** Lastly, the terminology used herein is for the purpose of describing particular embodiments only, and is not intended to limit the scope of systems, apparatuses, and methods as disclosed herein, which is defined solely by the claims. Accordingly, the systems, apparatuses, and methods are not limited to that precisely as shown and described.

Certain embodiments of systems, apparatuses, and methods are described herein, including the best mode known to the inventors for carrying out the same. Of course, variations on these described embodiments will become apparent to those of ordinary skill in the art upon reading the foregoing description. The inventor expects skilled artisans to employ such variations as appropriate, and the inventors intend for the systems, apparatuses, and methods to be practiced otherwise than specifically described herein. Accordingly, the systems, apparatuses, and methods include all modifications of the subject matter recited in the claims appended hereto as permitted by applicable law. Moreover, any combination of the above-described embodiments in all possible variations thereof is encompassed by the systems, apparatuses, and methods unless otherwise indicated herein or otherwise clearly contradicted by context.

Groupings of alternative embodiments, elements, or steps of the systems, apparatuses, and methods are not to be construed as limitation

Unless otherwise indicated, all numbers expressing a characteristic, item, quantity, parameter, property, term, and so forth used in the present specification and claims are to be understood as being modified in all instances by the term "about." As used herein, the term "about" means that the characteristic, item, quantity, parameter, property, or term so qualified encompasses an approximation that may vary, yet is capable of performing the desired operation or process discussed herein.

The terms "a," "an," "the" and similar referents used in the context of describing the systems, apparatuses, and methods (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the systems, apparatuses, and methods and does not pose a limitation on the scope of the systems, apparatuses, and methods otherwise claimed. No language in the present specification should be construed as indicating any non-claimed element essential to the practice of the systems, apparatuses, and methods.

## Claims

1. A deployment system for deploying a filter (70) in a subject, the deployment system comprising:
a deployment apparatus including an elongate shaft (24) having a deployment device;
a filter body (73) having a proximal portion (72) and a distal portion, and configured to have a deployed state in which the filter body (73) extends radially outward from the elongate shaft (24) and increases in size from the proximal portion (72) to the distal portion, the filter body (73) configured to trap particles in the filter body (73);
a filter support (68) being positioned distal of the proximal portion (72) of the filter body (73) and configured to couple to the elongate shaft (24) and slide relative to the filter body (73);
one or more support tethers (66) extending from the filter support (68) to the distal portion of the filter body (73); and
a control device (64) passing distally through the proximal portion (72) of the filter body and coupling to the filter support (68), the control device (64) configured to be slid proximally relative to the filter body (73) to slide the filter support (68) proximally to move the one or more support tethers (66) and transition the filter body (73) to the deployed state.

2. The deployment system of claim 1, wherein the control device (64) is configured to be slid distally relative to the filter body (73) to slide the filter support (68) distally to move the one or more support tethers (66) and transition the filter body (73) to an undeployed state.

3. The deployment system of claim 1 or claim 2, wherein the control device (64) is configured to be slid to control a size of the distal portion.

4. The deployment system of any of claims 1-3, wherein the control device (64) comprises a control tether.

5. The deployment system of any of claims 1-4, wherein the filter support (68) comprises a ring configured to extend around the elongate shaft (24).

6. The deployment system of any of claims 1-5, wherein the filter body (73):
a) has a conical shape in the deployed state, the conical shape increasing in size from the proximal portion (72) of the filter body (73) to the distal portion of the filter body (73); and/or
b) includes an opening of an interior cavity for retaining the particles, the opening being positioned at the distal portion of the filter body (73), and the filter body (73) extending around the interior cavity.

7. The deployment system of any of claims 1-6, wherein the filter body (73) is configured to slide relative to the elongate shaft (24).

8. The deployment system of any of claims 1-7, wherein the deployment device comprises one or more of an implant retention device or a dilation device, and the filter body (73) is configured to be positioned proximal of the deployment device.

9. A method for deploying a filter in a subject comprising:
inserting a filter within a subject, the filter positioned upon an elongate shaft (24) of a deployment apparatus having a deployment device, the filter including:
a filter body (73) having a proximal portion (72) and a distal portion, and configured to have a deployed state in which the filter body (73) extends radially outward from the elongate shaft (24) and increases in size from the proximal portion (72) to the distal portion, the filter body (73) configured to trap particles in the filter body (73),
a filter support (68) being positioned distal of the proximal portion (72) of the filter body (73) and on the elongate shaft (24) and configured to slide relative to the filter body (73),
one or more support tethers (66) extending from the filter support (68) to the distal portion of the filter body (73), and
sliding a control device (64), that passes distally through the proximal portion (72) of the filter body (73) and couples to the filter support (68), proximally to slide the filter support (68) proximally to move the one or more support tethers (66) and transition the filter body (73) to the deployed state,
wherein the subject comprises a cadaver or simulator of the cardiac and vasculature system.

10. The method of claim 9, wherein the filter is positioned within:
a) a blood vessel of the subject and is positioned proximal of a heart valve that the deployment apparatus is deploying a device to; and/or
b) an aortic arch proximal of an aortic valve.

11. The method of claim 9 or claim 10, wherein the deployment apparatus is deploying an implant to an aortic valve.

12. The method of any of claims 9-11, further comprising:
a) sliding the filter along the elongate shaft (24) of the deployment apparatus; and/or
b) sliding the control device (64) distally to slide the filter support (68) distally to move the one or more support tethers (66) and transition the filter body (73) to an undeployed state.

13. The method of any of claims 9-12, wherein the filter body (73) has a conical shape in the deployed state, the conical shape increasing in size from the proximal portion (72) of the filter body (73) to the distal portion of the filter body (73).

14. The method of any of claims 9-13, wherein the filter support (68) comprises a ring configured to extend around the elongate shaft (24).

15. The method of any of claims 9-14, wherein the control device (64) comprises a control tether.

## Patentansprüche

1. Einsetzsystem zum Einsetzen eines Filters (70) in ein Subjekt, wobei das Einsetzsystem umfasst:
ein Einsetzgerät, das einen länglichen Schaft (24) mit einer Einsetzvorrichtung einschließt;
einen Filterkörper (73) mit einem proximalen Abschnitt (72) und einem distalen Abschnitt, und der ausgestaltet ist, um einen eingesetzten Zustand zu haben, in dem der Filterkörper (73) sich von dem länglichen Schaft (24) radial auswärts erstreckt und in der Größe von dem proximalen Abschnitt (72) zu dem distalen Abschnitt zunimmt, wobei der Filterkörper (73) ausgestaltet ist, um Partikel in dem Filterkörper (73) einzufangen;
einen Filterträger (68), der distal von dem proximalen Abschnitt (72) des Filterkörpers (73) positioniert ist und ausgestaltet ist, um an den länglichen Schaft (24) zu koppeln und relativ zu dem Filterkörper (73) zu gleiten;
einen oder mehrere Tragestricke (66), die sich von dem Filterträger (68) zu dem distalen Abschnitt des Filterkörpers (73) erstrecken; und
eine Steuervorrichtung (64), die den proximalen Abschnitt (72) des Filterkörpers nach distal passiert und an den Filterträger (68) koppelt, wobei die Steuervorrichtung (64) ausgestaltet ist, um relativ zu dem Filterkörper (73) nach proximal geschoben zu werden, um den Filterträger (68) nach proximal zu schieben, um den einen oder die mehreren Tragestricke (66) zu bewegen und den Filterkörper (73) in den eingesetzten Zustand übergehen zu lassen.

2. Einsetzsystem nach Anspruch **1,** wobei die Steuervorrichtung (64) ausgestaltet ist, um relativ zu dem Filterkörper (73) nach distal geschoben zu werden, um den Filterträger (68) nach distal zu schieben, um den einen oder die mehreren Tragestricke (66) zu bewegen und den Filterkörper (73) in einen nicht-eingesetzten Zustand übergehen zu lassen.

3. Einsetzsystem nach Anspruch 1 oder Anspruch **2,** wobei die Steuervorrichtung (64) ausgestaltet ist, um geschoben zu werden, um eine Größe des distalen Abschnitts zu steuern.

4. Einsetzsystem nach einem der Ansprüche 1 bis **3,** wobei die Steuervorrichtung (64) einen Steuerstrick umfasst.

5. Einsetzsystem nach einem der Ansprüche 1 bis **4,** wobei der Filterträger (68) einen Ring umfasst, der ausgestaltet ist, um sich um den länglichen Schaft (24) zu erstrecken.

6. Einsetzsystem nach einem der Ansprüche 1 bis **5,** wobei der Filterkörper (73):
a) im eingesetzten Zustand eine konische Form hat, wobei die konische Form in der Größe von dem proximalen Abschnitt (72) des Filterkörpers (73) zu dem distalen Abschnitt des Filterkörpers (73) zunimmt; und/oder
b) eine Öffnung eines inneren Hohlraums zum Festhalten der Partikel einschließt, wobei die Öffnung an dem distalen Abschnitt des Filterkörpers (73) positioniert ist und der Filterkörper (73) sich um den inneren Hohlraum erstreckt.

7. Einsetzsystem nach einem der Ansprüche 1 bis 6, wobei der Filterträger (73) ausgestaltet ist, um relativ zu dem länglichen Schaft (24) zu gleiten.

8. Einsetzsystem nach einem der Ansprüche 1 bis 7, wobei die Einsetzvorrichtung eine oder mehrere von einer Implantatretentionsvorrichtung oder einer Dilationsvorrichtung umfasst, und der Filterkörper (73) ausgestaltet ist, um proximal zu der Einsetzvorrichtung positioniert zu werden.

9. Verfahren zum Einsetzen eines Filters in einem Subjekt, umfassend:
Einsetzen eines Filters innerhalb eines Subjekts, wobei der Filter auf einem länglichen Schaft (24) eines Einsetzgeräts, das eine Einsetzvorrichtung aufweist,
positioniert ist, wobei der Filter einschließt:
einen Filterkörper (73) mit einem proximalen Abschnitt (72) und einem distalen Abschnitt, und der ausgestaltet ist, um einen eingesetzten Zustand zu haben, in dem der Filterkörper (73) sich von dem länglichen Schaft (24) radial auswärts erstreckt und in der Größe von dem proximalen Abschnitt (72) zu dem distalen Abschnitt zunimmt, wobei der Filterkörper (73) ausgestaltet ist, um Partikel in dem Filterkörper (73) einzufangen,
einen Filterträger (68), der distal von dem proximalen Abschnitt (72) des Filterkörpers (73) und auf dem länglichen Schaft (24) positioniert ist und ausgestaltet ist, um relativ zu dem Filterkörper (73) zu gleiten,
einen oder mehrere Tragestricke (66), die sich von dem Filterträger (68) zu dem distalen Abschnitt des Filterkörpers (73) erstrecken, und
Schieben einer Steuervorrichtung (64), die den proximalen Abschnitt (72) des Filterkörpers (73) nach distal passiert und an den Filterträger (68) koppelt, nach proximal, um den Filterträger (68) nach proximal zu schieben, um den einen oder die mehreren Tragestricke (66) zu bewegen und den Filterkörper (73) in den eingesetzten Zustand übergehen zu lassen,
wobei das Subjekt einen Kadaver oder Simulator des Herz- und Gefäßsystems umfasst.

10. Verfahren nach Anspruch 9, wobei der Filter positioniert wird innerhalb von:
a) einem Blutgefäß des Subjekts, und proximal zu einer Herzklappe positioniert wird, worin das Einsetzgerät eine Vorrichtung einsetzt; und/oder
b) einem Aortenbogen proximal zu einer Aortenklappe.

11. Verfahren nach Anspruch 9 oder Anspruch 10, wobei das Einsetzgerät ein Implantat in eine Aortenklappe einsetzt.

12. Verfahren nach einem der Ansprüche 9 bis 11, des Weiteren umfassend:
a) Schieben des Filters entlang des länglichen Schafts (24) des Einsetzgeräts; und/oder
b) Schieben der Steuervorrichtung (64) nach distal, um den Filterträger (68) nach distal zu schieben, um den einen oder die mehreren Tragestricke (66) zu bewegen und den Filterkörper (73) in einen nicht-eingesetzten Zustand übergehen zu lassen.

13. Verfahren nach einem der Ansprüche 9 bis 12, wobei der Filterkörper (73) im eingesetzten Zustand eine konische Form hat, wobei die konische Form in der Größe von dem proximalen Abschnitt (72) des Filterkörpers (73) zu dem distalen Abschnitt des Filterkörpers (73) zunimmt.

14. Verfahren nach einem der Ansprüche 9 bis 13, wobei der Filterträger (68) einen Ring umfasst, der ausgestaltet ist, um sich um den länglichen Schaft (24) zu erstrecken.

15. Verfahren nach einem der Ansprüche 9 bis 14, wobei die Steuervorrichtung (64) einen Steuerstrick umfasst.

## Revendications

1. Système de déploiement pour déployer un filtre (70) dans un sujet, le système de déploiement comprenant :
un appareil de déploiement comprenant un arbre allongé (24) ayant un dispositif de déploiement ;
un corps de filtre (73) ayant une partie proximale (72) et une partie distale, et configuré pour avoir un état déployé dans lequel le corps de filtre (73) s'étend radialement vers l'extérieur à partir de l'arbre allongé (24) et augmente en taille de la partie proximale (72) à la partie distale, le corps de filtre (73) étant configuré pour piéger des particules dans le corps de filtre (73) ;
un support de filtre (68) étant positionné de manière distale à la partie proximale (72) du corps de filtre (73) et configuré pour se coupler à l'arbre allongé (24) et glisser par rapport au corps de filtre (73) ;
un ou plusieurs liens de support (66) s'étendant du support de filtre (68) à la partie distale du corps de filtre (73) ; et
un dispositif de commande (64) passant distalement à travers la partie proximale (72) du corps de filtre et se couplant au support de filtre (68), le dispositif de commande (64) étant configuré pour être glissé proximalement par rapport au corps de filtre (73) pour faire glisser le support de filtre (68) proximalement pour déplacer le ou les liens de support (66) et faire passer le corps de filtre (73) à l'état déployé.

2. Système de déploiement selon la revendication **1, le** dispositif de commande (64) étant configuré pour être glissé distalement par rapport au corps de filtre (73) pour faire glisser le support de filtre (68) distalement pour déplacer le ou les liens de support (66) et faire passer le corps de filtre (73) à un état non déployé.

3. Système de déploiement selon la revendication 1 ou selon la revendication **2,** le dispositif de commande (64) étant configuré pour être glissé pour commander une taille de la partie distale.

4. Système de déploiement selon l'une quelconque des revendications 1 à **3,** le dispositif de commande (64) comprenant un lien de commande.

5. Système de déploiement selon l'une quelconque des revendications 1 à 4, le support de filtre (68) comprenant un anneau configuré pour s'étendre autour de l'arbre allongé (24).

6. Système de déploiement selon l'une quelconque des revendications 1 à **5,** le corps de filtre (73) :
a) ayant une forme conique dans l'état déployé, la forme conique augmentant en taille de la partie proximale (72) du corps de filtre (73) à la partie distale du corps de filtre (73) ; et/ou
b) comprenant une ouverture d'une cavité intérieure pour retenir les particules, l'ouverture étant positionnée au niveau de la partie distale du corps de filtre (73), et le corps de filtre (73) s'étendant autour de la cavité intérieure.

7. Système de déploiement selon l'une quelconque des revendications 1 à 6, le corps de filtre (73) étant configuré pour glisser par rapport à l'arbre allongé (24).

8. Système de déploiement selon l'une quelconque des revendications 1 à 7, le dispositif de déploiement comprenant un ou plusieurs d'un dispositif de rétenue d'implant ou d'un dispositif de dilatation, et le corps de filtre (73) étant configuré pour être positionné de manière proximale au dispositif de déploiement.

9. Procédé pour déployer un filtre dans un sujet comprenant :
l'insertion d'un filtre dans un sujet, le filtre positionné sur un arbre allongé (24) d'un appareil de déploiement ayant un dispositif de déploiement, le filtre comprenant :
un corps de filtre (73) ayant une partie proximale (72) et une partie distale, et configuré pour avoir un état déployé dans lequel le corps de filtre (73) s'étend radialement vers l'extérieur à partir de l'arbre allongé (24) et augmente en taille de la partie proximale (72) à la partie distale, le corps de filtre (73) étant configuré pour piéger des particules dans le corps de filtre (73),
un support de filtre (68) étant positionné de manière distale à la partie proximale (72) du corps de filtre (73) et sur l'arbre allongé (24) et configuré pour glisser par rapport au corps de filtre (73),
un ou plusieurs liens de support (66) s'étendant du support de filtre (68) à la partie distale du corps de filtre (73), et
le glissement d'un dispositif de commande (64), qui passe distalement à travers la partie proximale (72) du corps de filtre (73) et se couple au support de filtre (68), proximalement pour faire glisser le support de filtre (68) proximalement pour déplacer le ou les liens de support (66) et faire passer le corps de filtre (73) à l'état déployé,
le sujet comprenant un cadavre ou un simulateur du système cardiaque et vasculaire.

10. Procédé selon la revendication 9, le filtre étant positionné dans :
a) un vaisseau sanguin du sujet et étant positionné de manière proximale à une valve cardiaque vers laquelle l'appareil de déploiement déploie un dispositif ; et/ou
b) un arc aortique proximal d'une valve aortique.

11. Procédé selon la revendication 9 ou selon la revendication 10, l'appareil de déploiement déployant un implant vers une valve aortique.

12. Procédé selon l'une quelconque des revendications 9 à 11, comprenant en outre :
a) le glissement du filtre le long de l'arbre allongé (24) de l'appareil de déploiement, et/ou
b) le glissement du dispositif de commande (64) distalement pour faire glisser le support de filtre (68) distalement pour déplacer le ou les liens de support (66) et faire passer le corps de filtre (73) à un état non déployé.

13. Procédé selon l'une quelconque des revendications 9 à 12, le corps de filtre (73) ayant une forme conique dans l'état déployé, la forme conique augmentant en taille de la partie proximale (72) du corps de filtre (73) à la partie distale du corps de filtre (73).

14. Procédé selon l'une quelconque des revendications 9 à 13, le support de filtre (68) comprenant un anneau configuré pour s'étendre autour de l'arbre allongé (24).

15. Procédé selon l'une quelconque des revendications 9 à 14, le dispositif de commande (64) comprenant un lien de commande.
